# EUROPEAN PATENT SPECIFICATION

(11) **EP 2 095 773 B1**
(45) Date of publication and mention of the grant of the patent: **25.05.2011**
(21) Application number: 09250532.0
(22) Date of filing: 26.02.2009
(51) Int. Cl.: A61B 10/02, A61M 25/00, A61B 17/34

(54) **Biopsy probe with hypodermic lumen**
Biopsiesonde mit Injektionslumen
Sonde de biopsie avec lumière hypodermique

(30) Priority: 27.02.2008 US 38180
(43) Date of publication of application: 02.09.2009
(73) Proprietor: Devicor Medical Products, Inc., Pleasant Prairie, WI 53158 (US)
(72) Inventor: Speeg, Trevor W.V., Williamsburg, OH 45176 (US); Reichel, Lee E., Springboro, OH 45066 (US); Leimbach, Jessica P., Cincinnati, OH 45209 (US); Johnson, Michael E., West Chester, OH 45069 (US)
(74) Representative: Tunstall, Christopher Stephen

(56) References cited:
- EP-A- 0 243 341
- EP-A- 1 579 810
- WO-A-92/04059
- GB-A- 2 323 283
- US-A1- 2004 191 897
- US-A1- 2006 224 082

## Description

### BACKGROUND

Biopsy samples have been obtained in a variety of ways in various medical procedures using a variety of devices. Biopsy devices may be used under stereotactic guidance, ultrasound guidance, MRI guidance, or otherwise. Merely exemplary biopsy devices are disclosed in U.S. Pat. No. 5,526,822, entitled "Method and Apparatus for Automated Biopsy and Collection of Soft Tissue," issued June 18, 1996; U.S. Pat. No. 6,086,544, entitled "Control Apparatus for an Automated Surgical Biopsy Device," issued July 11, 2000; U.S. Pub. No. 2003/0109803, entitled "MRI Compatible Surgical Biopsy Device," published June 12, 2003; U.S. Pub. No. 2007/0118048, entitled "Remote Thumbwheel for a Surgical Biopsy Device," published May 24, 2007; U.S. Provisional Patent Application Serial No. 60/869,736, entitled "Biopsy System," filed December 13, 2006; U.S. Provisional Patent Application Serial No. 60/874,792, entitled "Biopsy Sample Storage," filed December 13, 2006; and U.S. Non-Provisional Patent Application Serial No. 11/942,785, entitled "Revolving Tissue Sample Holder for Biopsy Device," filed November 21, 2007.

In some settings, a physician may wish to insert medication through a probe in a biopsy cavity. In some biopsy systems, this may require manipulation of various stopcocks, deactivating a vacuum, etc. In some (but not necessarily all) contexts, it may be desirable to be able to insert medication or perform other procedures through a biopsy probe without having to manipulate valves, deactivate otherwise active components, etc.

While several systems and methods have been made and used for obtaining a biopsy sample, it is believed that no one prior to the inventors has made or used the invention described in the appended claims.

EP 1 579 810 discloses a needle assembly comprising an upper cutter lumen and a lower vacuum lumen, in which a distal portion of the needle can include interlumen vacuum holes providing flow communication between the cutter lumen and the vacuum lumen. The preamble of claim 1 is based on this document.

### SUMMARY

The invention is defined by the independent claims. Optional features are included in the dependent claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements and in which:

FIG. 1 depicts a perspective view of an exemplary assembled biopsy device, for use in a stereotactic setting;

FIG. 2 depicts a perspective view of an exemplary assembled biopsy device, for use in an ultrasound setting;

FIG. 3 depicts a cross-sectional view of the distal end of an exemplary needle assembly, taken along a longitudinal plane;

FIG. 4 depicts a cross-sectional view of the needle assembly of FIG. 3, taken along a transverse plane;

FIG. 5 depicts an end view of the needle assembly of FIG. 3, showing the distal tip of the needle assembly;

FIG. 6 depicts a lateral plan view of the needle assembly of FIG. 3;

FIG. 7 depicts a top plan view of the needle assembly of FIG. 3;

FIG. 8 depicts an end view of the needle assembly of FIG. 3, showing a hub at the proximal end, with ports in partial cross-section;

FIG. 9 depicts a cross-sectional view of an exemplary alternative needle assembly, taken along a transverse plane;

FIG. 10 depicts a lateral plan view of an exemplary modification to the needle assembly of FIG. 3;

FIG. 11 depicts an end view of the needle assembly of FIG. 10, showing the distal tip of the needle assembly; and

FIG. 12 depicts an end view of the needle assembly of FIG. 11, showing the distal tip of the needle assembly, with a further modification.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

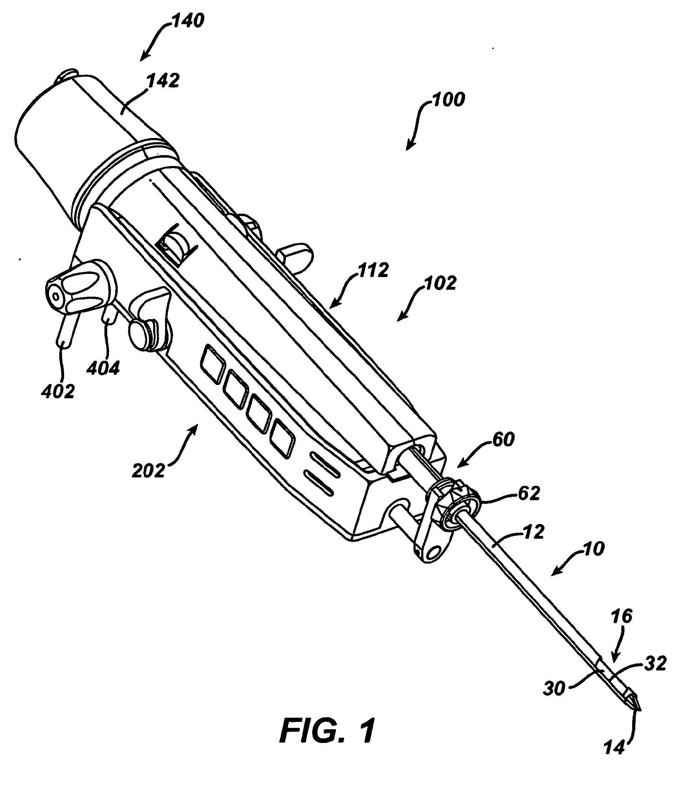

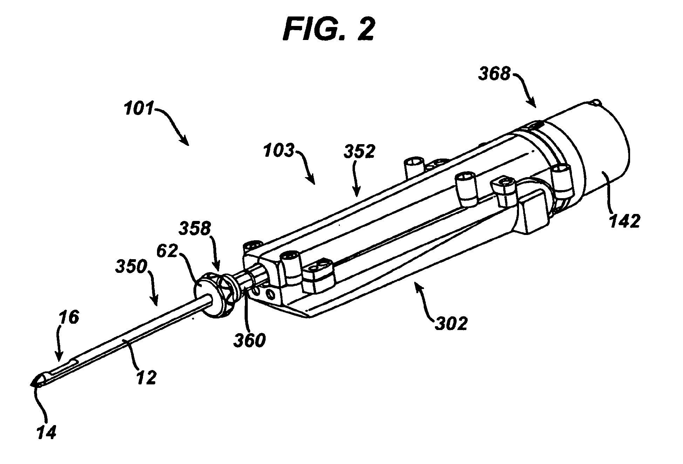

As shown in FIG. 1, an exemplary biopsy device (100) comprises a probe (102) and a holster (202). Similarly, as shown in FIG. 2, another biopsy device (101) comprises a probe (103) and a holster (302). Each probe (102, 103) is separable from its corresponding holster (202, 302). By way of example only, probe (102, 103) may be provided as a disposable component, while holster (202, 302) may be provided as a reusable component. Use of the term "holster" herein should not be read as requiring any portion of probe (102, 103) to be inserted into any portion of holster (202, 302). Indeed, in some variations of biopsy devices (100, 101), probe (102, 103) may simply sit on holster (202, 302). In some other variations, a portion of holster (202, 302) may be inserted into probe (102, 103). Furthermore, in some biopsy devices (100, 101), probe (102, 103) and holster (202, 302) may be of unitary or integral construction, such that the two components cannot be separated or are not identifiable as different components. Still other suitable structural and functional relationships between probe (102, 103) and holster (202, 302) will be apparent to those of ordinary skill in the art in view of the teachings herein.

I. Exemplary Biopsy Device for Stereotactic Use

As shown in FIG. 1, probe (102) comprises a needle portion (10) and a body portion (112). A tissue sample holder (140) is removably secured to body portion (112), though tissue sample holder (140) may alternatively be secured to some other component. A pair of tubes (402, 404) are coupled with probe (102) for communication of fluids (e.g., vacuum, saline, atmospheric air, pressurized air, etc.).

In the present example, needle portion (10) comprises an outer cannula (12) having a tissue piercing tip (14) and a transverse tissue receiving aperture (16). The interior of outer cannula (12) of the present example defines a cannula lumen (not shown) and a vacuum lumen (not shown), with a wall (30) separating the cannula lumen from the vacuum lumen. A plurality of external openings (not shown) are formed in outer cannula (12), and are in fluid communication with the vacuum lumen. Examples of openings that are similar to external openings (22) are disclosed in U.S. Pub. No. 2007/0032742, entitled "Biopsy Device with Vacuum Assisted Bleeding Control," published February 8, 2007. Of course, as with other components described herein, such external openings are merely optional. The wall (30) between the cannula lumen and the vacuum lumen also has a plurality of openings (32), permitting fluid communication between the cannula lumen and the vacuum lumen, though such openings are also merely optional.

A hollow cutter (not shown) is disposed within the cannula lumen of cannula (20). The interior of the cutter defines a cutter lumen, such that fluid and tissue may be communicated through the cutter via the cutter lumen. The cutter is configured to rotate within the cannula lumen and translate axially within the cannula lumen. Suitable mechanisms that may be provided for causing the cutter to rotate and translate are disclosed in U.S. Non-Provisional Patent Application Ser. No. 11/942,764, filed November 20, 2007, and entitled "Vacuum Timing Algorithm for Biopsy Device," while other suitable mechanisms will be apparent to those of ordinary skill in the art in view of the teachings herein. The cutter may be configured to sever a biopsy sample from tissue protruding through transverse aperture (16) of outer cannula (12), and may permit severed tissue samples to be communicated proximally through the cutter lumen. Merely illustrative examples of such severing and proximal communication are described in U.S. Pat. No. 5,526,822, though any other suitable structures or techniques may be used for severing and/or communicating tissue samples within a biopsy system.

As shown in FIG. 1, a needle hub (60) is secured to outer cannula (12), and comprises a thumbwheel (62) for rotating cannula (12), such as to reorient aperture (16) to capture several tissue samples during a single insertion of needle portion (10) as disclosed in U.S. Pat. No. 5,526,822. Needle hub (60) may include an interior portion that is in fluid communication with the vacuum lumen of outer cannula (12). Needle hub (60) may further be in fluid communication with a manifold (not shown) that is in further communication with either or both of tubes (402, 404). Suitable ways in which needle hub (60) may be in fluid communication with a lumen in outer cannula (12), as well as various features and configurations for needle hub (60), are disclosed in U.S. Non-Provisional Patent Application Ser. No. 11/942,764, filed November 20, 2007, and entitled "Vacuum Timing Algorithm for Biopsy Device."
Of course, any other suitable components, structures, or configurations may be used.

In the present example, a tissue sample holder (140) is provided at the proximal end of body portion (112) of probe (102). Tissue sample holder (140) comprises a cup (142), a rotatable manifold (not shown), and a plurality of removable sample trays (not shown) with a plurality of tissue sample chambers (not shown). Each tissue sample chamber is configured to separately hold a tissue sample communicated proximally through the cutter lumen, such that tissue sample holder (140) may separately hold a plurality of tissue samples. In particular, the manifold is configured to rotate to selectively index a tissue sample chamber relative to the cutter lumen. Manifold is further configured to communicate a vacuum from tube (404) to the cutter lumen, regardless of which tissue sample chamber is indexed relative to the cutter lumen. Suitable components and structures for and methods of operating a tissue sample holder (140) are disclosed in U.S. Non-Provisional Patent Application Ser. No. 11/942,764, filed November 20, 2007, and entitled "Vacuum Timing Algorithm for Biopsy Device." Of course, any other suitable components, structures, or configurations may be used.

Holster (202) of the present example comprises a needle rotation mechanism (not shown), a needle firing mechanism (not shown), a cutter drive mechanism (not shown), and a tissue holder rotation mechanism (not shown). The needle rotation mechanism is operable to rotate needle portion (10) about its longitudinal axis. The needle firing mechanism is operable to fire needle portion (10) into tissue. The cutter drive mechanism is operable to cause the cutter to rotate and translate; while the tissue holder rotation mechanism is operable to cause at least a portion of the tissue sample holder (140) to rotate. Suitable components and structures that may be used to provide any of these mechanisms, as well as other optional features of holster (202), are disclosed in U.S. Non-Provisional Patent Application Ser. No. 11/942,764, filed November 20, 2007, and entitled "Vacuum Timing Algorithm for Biopsy Device," Of course, any other suitable components, structures, or configurations may be used. Alternatively, any such mechanisms may simply be omitted altogether.

As noted above, holster (202) of the present example is configured to be coupled with a biopsy probe (102), such as biopsy probe (102) described above, to provide a biopsy device (100). In addition, holster (202) is configured to be mounted to a table, fixture, or other device, such as for use in a stereotactic or X-ray setting. However, it will be appreciated in view of the disclosure herein that holster (202) may be used in a variety of other settings and combinations.

II. Exemplary Biopsy Device for Ultrasound Use

As shown in FIG. 2, an alternative biopsy probe (103) comprises a needle portion (350) and a body portion (352). A tissue sample holder (368) is removably secured to the body portion, though tissue sample holder (368) may alternatively be secured to some other component. A pair of tubes (not shown) are coupled with probe (103). As will also be described in greater detail below, and as noted above, biopsy probe (103) is configured to be coupled with a holster (302) to provide a biopsy device (101).

In the present example, needle portion (350) comprises an outer cannula (12) having a tissue piercing tip (14) and a transverse tissue receiving aperture (16) located proximally from the tissue piercing tip (14). In this example, these components are essentially the same as the components bearing the same names and item numbers described above, so they will not be described in greater detail here. In other words, the features, properties, and components of outer cannula (12), tip (14), and aperture (16) as described above (including the cannula lumen, the vacuum lumen, wall (30), transverse openings (32), etc.) may be the same for needle portion (350) as they were described above with respect to needle portion (10). Of course, they may alternatively be varied in any suitable way, as desired.

A hollow cutter in probe (103) may have the same relationship with needle portion (350) as the relationship described above between the cutter and needle portion (10); as well as all the same features, properties, and components as the cutter described above in the context of probe (102). Such aspects of the cutter will therefore not be repeated here. Alternatively, a cutter used with either probe (102, 103) may have any other features, properties, components, or relationships with needle portion (10) as desired.

A needle hub (358) is secured to outer cannula (12) of probe (103), and comprises a thumbwheel (62) and a sleeve portion (360) extending proximally from thumbwheel (62). Needle hub (358) of the present example is substantially similar to needle hub (60) described above, and will therefore not be described in greater detail here. Other optional features, components, and configurations for needle hub (358) are disclosed in U.S. Non-Provisional Patent Application Ser. No. 11/942,764, filed November 20, 2007, and entitled "Vacuum Timing Algorithm for Biopsy Device." Of course, any other suitable components, structures, or configurations may be used for needle hub (358).

In addition, a tissue sample holder (368) of probe (103) may be the same as or similar to tissue sample holder (140) described above. Alternatively, tissue sample holder (368) may include any tissue sample holder described in U.S. Non-Provisional Patent Application Scr. No. 11/942,764, filed November 20, 2007, and entitled "Vacuum Timing Algorithm for Biopsy Device." Of course, any other suitable components, structures, or configurations may be used for tissue sample holder (368), to the extent that any tissue sample holder is used at all.

As shown in FIG. 2, an alternative holster (302) comprises a cutter drive mechanism (not shown) and a tissue holder rotation mechanism (not shown). The cutter drive mechanism is operable to cause the cutter to rotate and translate; while the tissue holder rotation mechanism is operable to cause at least a portion of the tissue sample holder (368) to rotate. Suitable components and structures that may be used to provide any of these mechanisms, as well as other optional features of holster (202), are disclosed in U.S. Non-Provisional Patent Application Ser. No. 11/942,764, filed November 20, 2007, and entitled "Vacuum Timing Algorithm for Biopsy Device." Alternatively, any other suitable components, structures, or configurations may be used, or such mechanisms may simply be omitted altogether.

Holster (302) may further include a user interface that permits a user to enter commands to operate at least a portion of biopsy device (101). Suitable user interfaces that may be so incorporated into holster (302) are disclosed in U.S. Non-Provisional Patent Application Ser. No. 11/942,764, filed November 20, 2007, and entitled "Vacuum Timing Algorithm for Biopsy Device." Of course, any other suitable components, structures, or configurations may be used. Alternatively, holster (302) may simply lack a user interface altogether.

Holster (302) of the present example is configured to be coupled with a biopsy probe (103), such as biopsy probe (103) described above, to provide a biopsy device (101). In addition, holster (302) is configured to be handheld, such that biopsy device (101) may be manipulated and operated by a single hand of a user (e.g., using ultrasound guidance, etc.). However, it will be appreciated in view of the disclosure herein that holster (302) may be used in a variety of other settings and combinations. By way of example only, holster (302) may alternatively be coupled with biopsy probe (102) instead of biopsy probe (103). As another merely illustrative example, holster (302) may be coupled with a variation of biopsy probe (102) that has a modified needle hub (60) (e.g., a needle hub (60) that is shorter, not configured for firing needle portion (10), etc.).

III. Vacuum and Control

Either biopsy device (100, 101) may be coupled with a vacuum control module (not shown) that is operable to provide fluids (e.g., vacuum, atmospheric air, saline, pressurized air, etc.), power, and/or commands to biopsy device (100, 101). Suitable examples of such a vacuum control module are disclosed in U.S. Non-Provisional Patent Application Ser. No. 11/942,764, filed November 20, 2007, and entitled "Vacuum Timing Algorithm for Biopsy Device." Alternatively, any other suitable components, structures, or configurations may be used.

In other versions, biopsy device (100, 101) may be provided and used without a vacuum control module. By way of example only, biopsy device (100, 101) may have an on-board vacuum pump (not shown) and/or pressure pump (not shown). Merely exemplary biopsy devices with such on-board pumps are disclosed in U.S. Non-Provisional Patent Application Ser. No. 11/965,048, filed December 27, 2007, entitled "Vacuum Sensor and Pressure Pump for Tetherless Biopsy Device," and in U.S. Non-Provisional Patent Application Ser. No. 11/964,811, filed December 27, 2007, entitled "Clutch and Valving System for Tetherless Biopsy Device." Again, though, any other suitable components, structures, or configurations may be used.

IV. Exemplary Modified Needle Assembly

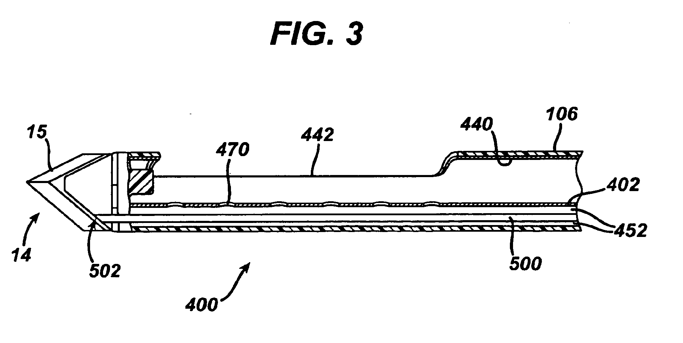

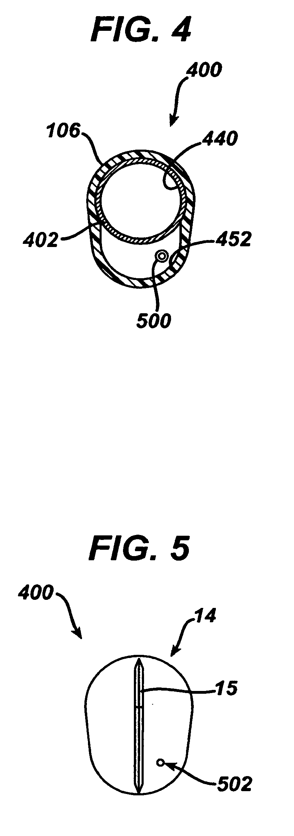

Either biopsy device (100, 101) described above may be modified to include a different needle assembly (400) instead of needle portion (10, 350). Suitable ways in which needle assembly (400) may be incorporated into either biopsy device (100, 101) will be apparent to those of ordinary skill in the art in view of the teachings herein. Views of an exemplary needle assembly (400) are shown in FIGS. 3-9, though many variations of needle assembly (400) may look significantly different from the merely illustrative versions shown in the drawings. In addition, while a medicine lumen (500) will be described below in the context of a needle assembly (400) that has a molded sheath (106), it will be appreciated that the medicine lumen (500) described herein may be incorporated into a variety of other needle assemblies, including but not limited to those that have no molded material (e.g., those made from extruded metal, etc.), and either of needle portions (10, 350) described above, among others.

Needle assembly (400) of this example is formed in part according to the processes described in, and may be used in accordance with uses described in, U.S. Pat. App. Pub. No. 2006/0144548, entitled "Method of Manufacturing a Needle Assembly for Use with a Biopsy Device." In particular, and as shown in FIGS. 3-4, needle assembly (400) comprises a tube (402) defining a cutter lumen (440), which is configured to receive a hollow cutter (not shown). Such a cutter may rotate and translate within cutter lumen (440). Tube (402) may be preformed, and may be substantially straight, substantially round, and substantially rigid. In some versions, tube (402) is formed of metal, such as titanium, titanium alloy, aluminum, or aluminum alloy, though any suitable material(s) may be used, including but not limited to plastics or other non-metallics or combinations thereof. A tissue-receiving port (442) may be formed transversely through tube (402), at a distal portion of tube (402). Cutter lumen (440) may also comprise an open proximal end and an open distal end.

Needle assembly (400) of the present example further comprises a vacuum lumen (452). In some versions, cutter lumen (440) is located above vacuum lumen (452). A vacuum source (not shown) may be in communication with vacuum lumen (452), possibly at proximal portion thereof, via a vacuum line or other component.

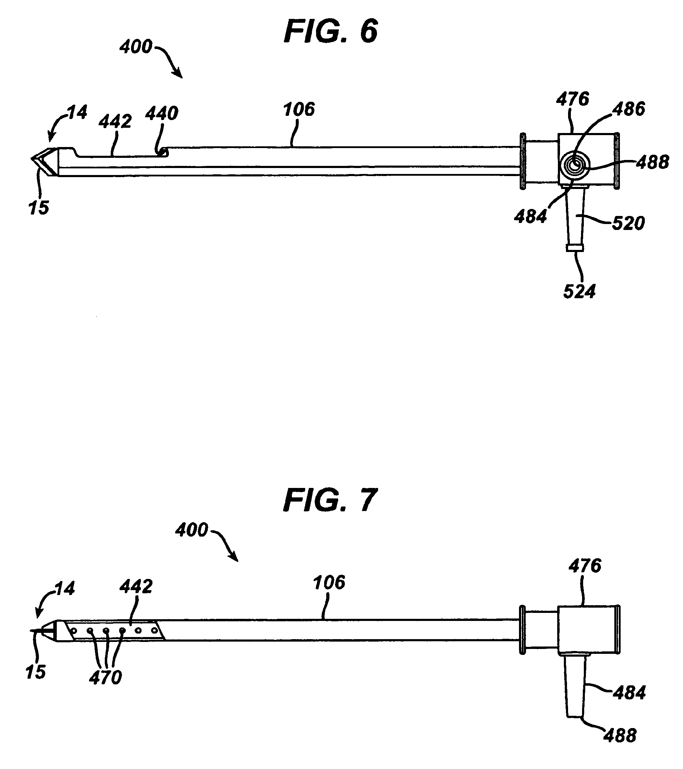

Needle assembly (400) may also include one or more passages or interlumen vacuum holes (470), between cutter lumen (440) and vacuum lumen (452). When a vacuum source is activated, thereby providing suction in vacuum lumen (452), interlumen vacuum holes (470) may allow that suction to be communicated into cutter lumen (440). As best illustrated in FIGS. 3 and 7, interlumen vacuum holes (470) may be located between cutter lumen (440) and vacuum lumen (452) opposite the tissue-receiving port (442).

A tissue piercing tip (14) having a sharp blade (15) is provided at the distal end of needle assembly (400). Tip (14) and/or blade (15) may be formed of a material having sufficient strength and rigidity to allow it to move through tissue with minimal, if any, deflection. For instance, tip (14) and/or blade (15) may be formed of stainless steel, titanium, titanium alloy, aluminum, or aluminum alloy. Non-metallic materials, such as ceramics, glass, and/or as MRI compatible resins, including but not limited to Ultem and Vectra, may also be used. Tip (14) and/or blade (15) may be welded or otherwise secured relative to tube (402).

Other features of needle assembly (400), including but not limited to vacuum lumen (452), may be formed by applying a coating of material over tube (402). The coating of material may be applied to tube (402) as a liquid, and then hardened to the necessary rigidity for use in the human body after formation of the desired features thereon. In some versions, the coating of material may be applied to tube (402) by injection molding. By way of example only, a mold (not pictured) may be designed such that the injected material may flow into predetermined cavities and form the desired features over tube (402), including but not limited to vacuum lumen (452) and hub (476), which will be described in greater detail below. One or more gates (not pictured) through which the material is injected into the mold may be positioned at any suitable location(s).

In the present example, material that is injected into a mold forms an outer sheath (106) over tube (402). A portion of the interior of sheath (106) and a portion of the exterior of tube (402) together define vacuum lumen (452) in this example. In some other versions, vacuum lumen (452) is drilled out of sheath (106) after sheath (106) has been molded about tube (402) and reaches sufficient hardness. Alternatively, vacuum lumen (452) may be formed using a variety of other components or techniques.

To the extent that an injection molding process is used to form at least a portion of needle assembly (400), the injected material may be selected from materials including, but not limited to, plastics, thermoplastics, thermoresins, and polymers. For instance, the molded features may be formed of a liquid crystal polymer or a glass reinforced polymer. One suitable material may include a glass reinforced liquid crystal polymer such as VECTRA A130 available from Ticona Corp. Alternatively, any other suitable material(s) having any suitable properties may be used. Furthermore, any process other than molding - injection or otherwise - may be used to form any or all of needle assembly (400).

As also shown in FIGS. 3-5, a medicine lumen (500) extends longitudinally through needle assembly (400). While FIG. 3 shows a lateral cross-section of a portion of needle assembly (400), it should be understood that medicine lumen (500) is still depicted in the drawing in non-cross-sectional form, despite the omission of other components of needle assembly (400) that are on the same side of needle assembly (400) as medicine lumen (500). Medicine lumen (500) is positioned in vacuum lumen (452), but is fluidly isolated from vacuum lumen (452), in this example. The distal end of medicine lumen (500) terminates in tip (14). In particular, a medicine opening (502) is formed in tip (14), which allows medicine that is communicated through medicine lumen (500) to be further communicated out of tip (14), through opening (502), to a site within a patient, such as a biopsy site. In the present example, medicine opening (502) is configured such that its presence will not have a significant adverse effect on the force that is required for needle assembly (400) to penetrate tissue. In other versions, opening (502) is located elsewhere, such as proximal to tip (14).

In some versions, medicine lumen (500) is formed by a tube (e.g., a metal tube, etc.) that is inserted through vacuum lumen (452) after vacuum lumen (452) has been formed. Such a tube may extend into tip (14), such as through a lumen formed in tip (14), and terminate at the distal face of tip (14). Alternatively, such a tube may terminate somewhere proximal to the distal face of tip (14) (e.g.,. within tip (14), at a proximal face of tip (14), etc.), with fluid communication continuing through a lumen formed in tip (14). Other suitable relationships between medicine lumen (500) and tip (14) will be apparent to those or ordinary skill in the art in view of the teachings herein.

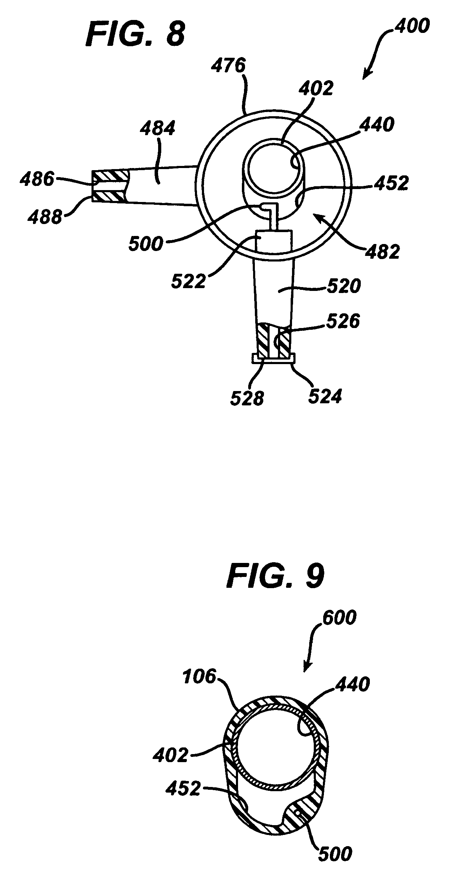

In other versions, vacuum lumen (452) is formed around medicine lumen (500). For instance, medicine lumen (500) may be provided by a tube, and outer sheath (106) may be molded around tube (402) and a medicine lumen (500) tube to form vacuum lumen (452), with medicine lumen (500) tube being spaced away from the interior wall of sheath (106) defining vacuum lumen (452). In still other versions, medicine lumen (500) is molded as a unitary part of outer sheath (106), as will be described in greater detail below with reference to FIG. 9. Other ways in which medicine lumen (500) may be formed will be apparent to those of ordinary skill in the art in view of the teachings herein.

As illustrated in FIGS. 6-8, a hub (476) may be located on the proximal portion of needle assembly (400). In some versions, hub (476) is formed by a molding process, such as the molding process described above. For instance, hub (476) and sheath (106) may be molded together (e.g., as a unitary construction); or they may be molded separately and later joined using any suitable techniques. Hub (476) may assist in mounting needle assembly (400) to a probe (102, 103) or other any other suitable support.

Hub (476) of the present example comprises a vacuum port (484) and a medicine port (520), each of which will be described in greater detail below. While vacuum port (484) is shown as extending laterally from hub (476) at a 9 o'clock position, it will be appreciated that vacuum port (484) may be provided at any other suitable orientation about hub (476). For instance, vacuum port (484) may extend at any other orientation lateral to hub (476), may extend longitudinally from hub (476), or may have any other suitable relationship with hub (476). Similarly, while medicine port (520) is shown as extending from hub (476) at a 6 o'clock position, medicine port (520) may alternatively be provided at any other suitable orientation about hub (476). For instance, medicine port (520) may extend at any other orientation lateral to hub (476), may extend longitudinally from hub (476), or may have any other suitable relationship with hub (476).

Hub (476) further defines an interior (482), which provides fluid communication from vacuum port (484) to vacuum lumen (452). Hub (476) thus provides a manifold in fluid communication with vacuum lumen (452). As shown in FIG. 8, vacuum port (484) defines a vacuum port lumen (486), which extends from an end (488) of vacuum port (484) to interior (484) of hub (476). Vacuum port (484) is configured to be coupled with a conduit (not shown) and/or a vacuum source (not shown) for communicating a vacuum or other fluid to interior (484), and therefore to vacuum lumen (452), via vacuum port lumen (486). It will be appreciated, however, that vacuum or other fluid may be communicated to vacuum lumen (452) using a variety of other structures and techniques. Accordingly, like all other components described herein, vacuum port (484) is merely optional. Furthermore, vacuum lumen (452) may be omitted altogether in some versions. Hub (476) may also provide a conduit for communication of a vacuum to cutter lumen (440). Suitable structures, configurations, and techniques for providing communication of a vacuum or other fluids to cutter lumen (440) will be apparent to those of ordinary skill in the art in view of the teachings herein.

The proximal end of medicine lumen (500) terminates in or at hub (476) in this example. In particular, hub (476) includes a medicine port (520), which is in fluid communication with medicine lumen (500) via a coupling (522). As shown in FIG. 8, medicine port (520) defines a medicine port lumen (526), which extends from an end (528) of medicine port (520) to coupling (522). Coupling (522) is fluidly isolated from interior (484) of hub (476). Medicine or other fluid may therefore be communicated from medicine port (520) to medicine lumen (500) without experiencing any effects from a vacuum in interior (484) or vacuum lumen (452) in this example.

In some versions, medicine port (520) includes a luer lock feature (not shown), such that a syringe or other component may be easily coupled with medicine port (520). For instance, a syringe or other component may be coupled with medicine port (520) to provide medicine to a biopsy site via medicine lumen (500) and opening (502), without necessarily interrupting a biopsy procedure, and without necessarily having to withdraw or relocate needle assembly (400) from or within the patient's tissue, before, during, and/or after a biopsy sample is acquired through needle assembly (400).

In some other versions, medicine port (520) comprises a rubber cap (524) (e.g., similar to a plastic needle entry port on a saline bag, etc.), which provides a septum. In such versions, a user may insert the needle of a syringe or some other component through cap (524) to inject medicine into medicine lumen (500). When the user withdraws the needle from cap (524), cap (524) may reseal to prevent medicine, blood, or other fluids from escaping medicine port (520). As an alternative to a cap (524), port (520) may include a plug (e.g., a rubber plug, etc.) or other feature. Other features that may be provided at, on, or in medicine port (520) will be apparent to those of ordinary skill in the art in view of the teachings herein.

Port (520) may also be provided at a variety of orientations relative to needle assembly (400). In other words, port (520) need not necessarily be substantially perpendicular to needle assembly (400) as shown in FIGS. 6 and 8. For instance, port (520) may be oriented at any desired angle relative to the axis defined by needle assembly (400), such as to provide convenience to the user. Furthermore, port (520) need not necessarily be provided as a component of hub (476). For instance, port (520) may be positioned at any suitable location on or near probe (102, 103), such as on or in body portion (112, 352). Port (520) and probe (102, 103) may be configured such that port (520) "blends in" with body portion (112, 352). Furthermore, port (520) may be selectively covered/uncovered by a small door or other feature of body portion (112, 352). Such a door or other feature may substantially follow the shape or contour of the body portion (112, 352). In still other versions, the outer surface of body portion (112, 352) and the outer surface of cap (524) are substantially coplanar or appear substantially continuous. Still other suitable relationships between, and relative configurations for, a port (520) and a probe (102, 103) will be apparent to those of ordinary skill in the art in view of the teachings herein.

An alternative needle assembly (600) is shown in FIG. 9. Needle assembly (600) of this example is substantially identical to needle assembly (400) described above. However, rather than medicine lumen (500) being defined by a tube, medicine lumen (500) is molded as part of sheath (106). In other words, medicine lumen (500) is defined by sheath (106) in this example. Medicine lumen (500) is still fluidly isolated from vacuum lumen (452), and is still in fluid communication with opening (502) and medicine port (520). Suitable methods for molding or otherwise creating medicine lumen (500) as part of sheath (106) will be apparent to those of ordinary skill in the art in view of the teachings herein. Similarly, other ways in which a medicine lumen (500) may be created, defined, and incorporated into a needle assembly (400, 600) will be apparent to those of ordinary skill in the art in view of the teachings herein.

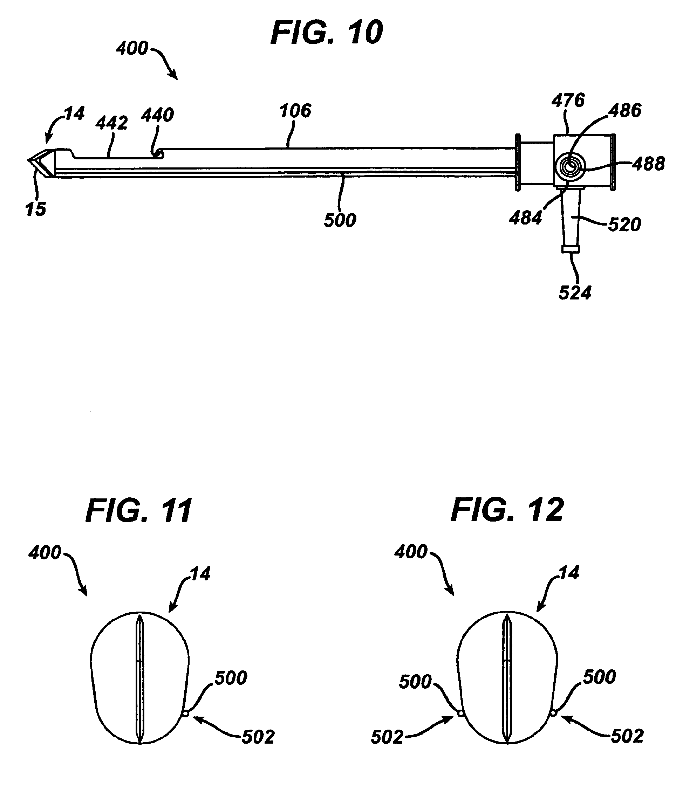

Further merely exemplary variations of needle assembly (400) are shown in FIGS. 10-12. As shown in FIGS. 10-11, medicine lumen (500) is positioned external to the outer wall of sheath (106) in this example. In particular, medicine lumen (500) runs external to yet adjacent to, and parallel with, sheath (106) in this example. Medicine lumen (500) terminates in an opening (502) located at or near tip (14) in this example, though opening (502) may be positioned at any other suitable location. In some other versions, a portion of medicine lumen (500) is external to sheath (106), protrudes relative to sheath (106), or causes a portion of sheath (106) to protrude, while another portion of medicine lumen (500) is internal to or internal relative to sheath (106). Other suitable relationships between medicine lumen (500) and sheath (106) will be apparent to those of ordinary skill in the art in view of the teachings herein.

It will also be appreciated in view of the teachings herein that more than one medicine lumen (500) may be provided, if desired. For instance, as shown in FIG. 12, a pair of medicine lumens (500) may be provided. In the example shown in FIG. 12, medicine lumens (500) are positioned on opposite sides of sheath (106), and both medicine lumens (500) terminate at respective openings (502) that are both located at substantially the same longitudinal position along sheath (106) at or near tip (14). However, in other versions, medicine lumens (500) may have any other suitable positioning about or relative to sheath (106) and need not be positioned opposite one another. Similarly, openings (502) of medicine lumens (500) may be positioned at different locations, and need not be both located at substantially the same longitudinal position along sheath (106), let alone at or near tip (14). Furthermore, while a pair of medicine lumens (500) are shown in FIG. 12, any other suitable number of medicine lumens (500) may be provided, including more than two or less than two.

To the extent that more than one medicine lumen (500) is provided, such a plurality of medicine lumens (500) may be in communication with a common port (520). For instance, a plurality of medicine lumens (500) may simultaneously be in communication with a common port (520). Alternatively, one or more valves or other features may be provided to provide selective communication of fluids from a common port (520) to one or more medicine lumens (500). In still other versions, each medicine lumen (500) of a plurality of medicine lumens (500) is in communication with its own dedicated port (520). Still other configurations for providing fluid communication, selective or otherwise, from one or more ports (520) to one or more medicine lumens (500) will be apparent to those of ordinary skill in the art in view of the teachings herein.

Suitable medicines that may be dispensed through medicine lumen (500) and opening (502) may include, by way of example only, Lidocane or other local anesthesia. While exemplary uses described herein include the dispensation of medicine through medicine lumen (500) and opening (502), it will be appreciated that a variety of other fluids or materials may be communicated through medicine lumen (500) and opening (502). For instance, a radiopaque material may be communicated through medicine lumen (500) and opening (502), such as to mark a biopsy site for future reference. Alternatively, any or all of the following may be communicated through medicine lumen (500) and opening (502), though this list is not intended to be exhaustive: saline for flushing, hemostatic agents, imaging enhancing agents, staining agents, brachytherapy components, blood or other fluids being sucked proximally therethrough from a biopsy site, etc. Still other materials that may be communicated through medicine lumen (500) and opening (502) will be apparent to those of ordinary skill in the art in view of the teachings herein.

Embodiments of the present invention have application in conventional endoscopic and open surgical instrumentation as well as application in robotic-assisted surgery.

Embodiments of the devices disclosed herein can be designed to be disposed of after a single use, or they can be designed to be used multiple times. Embodiments may, in either or both cases, be reconditioned for reuse after at least one use. Reconditioning may include any combination of the steps of disassembly of the device, followed by cleaning or replacement of particular pieces, and subsequent reassembly. In particular, embodiments of the device may be disassembled, and any number of the particular pieces or parts of the device may be selectively replaced or removed in any combination. Upon cleaning and/or replacement of particular parts, embodiments of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, embodiments described herein may be processed before surgery. First, a new or used instrument may be obtained and if necessary cleaned. The instrument may then be sterilized. In one sterilization technique, the instrument is placed in a closed an sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta or gamma radiation, ethylene oxide, or steam.

Having shown and described various embodiments of the present invention, further adaptations of the systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A biopsy probe (102: 103), wherein the biopsy probe comprises:
(a) a body portion (112; 352);
(b) a cannula (12) extending relative to the body portion, wherein the cannula comprises:
(i) a first lumen (440) configured to receive a cutter, and
(ii) a transverse aperture (442) configured to receive tissue;
(c) a tip (14) located at the distal end of the cannula, wherein the tip has an opening (502) formed therein;
(d) a cutter configured to translate relative to the cannula, wherein the cutter is disposed in the first lumen; and
(e) a second lumen (500) extending through the cannula, wherein the second lumen is in fluid communication with the opening formed in the tip, **characterised in that** the second lumen is fluidly isolated from the first lumen.

2. The biopsy probe (102; 103) of claim 1, wherein the cannula (12) further comprises a third lumen (452), wherein the third lumen is in fluid communication with the first lumen (440).

3. The biopsy probe (102; 103) of claim 2, wherein the cannula (12) further comprises one or more transverse openings (470) providing fluid communication from the third lumen (452) to the first lumen (440).

4. The biopsy probe (102; 103) of claim 2, wherein the cannula (12) further comprises:
(i) a tube (402) having an interior and an exterior, wherein the interior of the tube defines the first lumen (440), and
(ii) a sheath (106) overmolded about the tube, wherein a portion of the exterior of the tube and a portion of the sheath together define the third lumen (452).

5. The biopsy probe (102; 103) of claim 4, wherein the sheath (106) further defines the second lumen (500).

6. The biopsy probe (102; 103) of claim 2, further comprising a tube extending through the third lumen (452), wherein the tube defines the second lumen (500).

7. The biopsy probe (102; 103) of claim 1, further comprising a hub (476) secured relative to the cannula (12), wherein the hub comprises a first port (520) in fluid communication with the second lumen (500).

8. The biopsy probe (102; 103) of claim 7, wherein the first port (520) comprises a needle penetrable septum (524).

9. The biopsy probe (102; 103) of claim 7, wherein the hub (476) further comprises a second port (484) in fluid communication with at least a portion of the cannula (12).

10. The biopsy probe (102; 103) of claim 9, wherein the first port (520) and the second port (484) are fluidly isolated from one another.

11. The biopsy probe (102; 103) of claim 7, further comprising a coupling (522) between the first port (520) and the second lumen (500).

12. The biopsy probe (102; 103) of claim 7, wherein the cannula (12) defines an axis, wherein the first port (520) extends transversely relative to the axis.

13. The biopsy probe (102; 103) of claim 2, wherein the third lumen (452) is substantially parallel to the first lumen (440).

## Patentansprüche

1. Biopsiesonde (102; 103), wobei die Biopsiesonde
(a) einen Massekörper (112; 352);
(b) eine Kanüle (12), die sich relativ zum Massekörper ausdehnt, wobei die Kanüle
(i) ein erstes Lumen (440), das so gestaltet ist, einen Schneider aufzunehmen, und
(ii) eine querlaufende Apertur (442) umfasst, die so gestaltet ist, ein Gewebe aufzunehmen;
(c) eine Spitze (14), die an dem distalen Ende der Kanüle angebracht ist, wobei die Spitze eine darin geformte Öffnung (502) aufweist;
(d) einen Schneider, der so gestaltet ist, relativ zu der Kanüle verschoben zu werden, wobei der Schneider an dem ersten Lumen angebracht ist; und
(e) ein zweites Lumen (500) umfasst, das durch die Kanüle hindurchragt, wobei das zweite Lumen in Flüssigkeitsverbindung mit der in der Spitze gebildeten Öffnung steht, **dadurch gekennzeichnet, dass** das zweite Lumen von dem ersten Lumen flüssigkeitsbezogen isoliert ist.

2. Biopsiesonde (102; 103) nach Anspruch 1, wobei die Kanüle (12) ferner ein drittes Lumen (452) umfasst, wobei das dritte Lumen in Flüssigkeitsverbindung mit dem ersten Lumen (440) steht.

3. Biopsiesonde (102; 103) nach Anspruch 2, wobei die Kanüle (12) ferner eine oder mehrere querliegende Öffnungen (470) umfasst, die eine Flüssigkeitsverbindung von dem dritten Lumen (452) zu dem ersten Lumen (440) gewährleisten.

4. Biopsiesonde (102; 103) nach Anspruch 2, wobei die Kanüle (12) ferner
(i) eine Röhre (402) mit einem Inneren und einem Äußeren, wobei das Innere der Röhre das erste Lumen (440) abgrenzt, und
(ii) einen Mantel (106) umfasst, der die Röhre umgibt, wobei ein Teil des Äußeren der Röhre und ein Teil des Mantels zusammen das dritte Lumen (452) abgrenzt.

5. Biopsiesonde (102; 103) nach Anspruch 4, wobei der Mantel (106) ferner das zweite Lumen (500) abgrenzt.

6. Biopsiesonde (102; 103) nach Anspruch 2, die ferner eine Röhre umfasst, die durch das dritte Lumen (452) hindurchragt, wobei die Röhre das zweite Lumen (500) abgrenzt.

7. Biopsiesonde (102; 103) nach Anspruch 1, die ferner einen Ansatz (476) umfasst, der realtiv zu der Kanüle (12) befestigt ist, wobei der Ansatz einen ersten Anschluss (520) umfasst, der sich in Flüssigkeitsverbindung mit dem zweiten Lumen (500) befindet.

8. Biopsiesonde (102; 103) nach Anspruch 7, wobei der erste Anschluss (520) ein nadelpenetrierbares Septum (524) umfasst.

9. Biopsiesonde (102; 103) nach Anspruch 7, wobei der Ansatz (476) ferner einen zweiten Anschluss (484) in Flüssigkeitsverbindung mit mindestens einem Teil der Kanüle (12) umfasst.

10. Biopsiesonde (102; 103) nach Anspruch 9, wobei der erste Anschluss (520) und der zweite Anschluss (484) flüssigkeitsbezogen voneinander getrennt sind.

11. Biopsiesonde (102; 103) nach Anspruch 7, die ferner eine Kupplung (522) zwischen dem ersten Anschluss (520) und dem zweiten Lumen (500) umfasst.

12. Biopsiesonde (102; 103) nach Anspruch 7, wobei die Kanüle (12) eine Achse begrenzt, wobei der erste Anschluss (520) sich relativ zu der Achse querliegend ausdehnt.

13. Biopsiesonde (102; 103) nach Anspruch 2, wobei das dritte Lumen (452) im Wesentlichen parallel zu dem ersten Lumen (440) liegt.

## Revendications

1. Sonde de biopsie (102 ; 103), dans laquelle la sonde de biopsie comprend :
(a) une partie de corps (112 ; 352) ;
(b) une canule (12) qui s'étend par rapport à la partie de corps, dans laquelle la canule comprend :
(i) une première lumière (440) configurée de façon à recevoir un dispositif tranchant ; et
(ii) une ouverture transversale (442) configurée de façon à recevoir un tissu ;
(c) un bout (14) situé au niveau de l'extrémité distale de la canule, dans laquelle le bout présente une ouverture (502) formée à l'intérieur ;
(d) un dispositif tranchant configuré de façon à se translater par rapport à la canule, dans laquelle le dispositif tranchant est disposé dans la première lumière ; et
(e) une deuxième lumière (500) qui s'étend à travers la canule, dans laquelle la deuxième lumière est en communication fluidique avec l'ouverture formée dans le bout ;
**caractérisée en ce que** la deuxième lumière est isolée de manière fluidique de la première lumière.

2. Sonde de biopsie (102 ; 103) selon la revendication 1, dans laquelle la canule (12) comprend en outre une troisième lumière (452), dans laquelle la troisième lumière est en communication fluidique avec la première lumière (440).

3. Sonde de biopsie (102 ; 103) selon la revendication 2, dans laquelle la canule (12) comprend en outre une ou plusieurs ouvertures transversales (470) qui fournissent une communication fluidique à partir de la troisième lumière (452) vers la première lumière (440).

4. Sonde de biopsie (102 ; 103) selon la revendication 2, dans laquelle la canule (12) comprend en outre :
(i) un tube (402) qui présente un intérieur et un extérieur, dans laquelle l'intérieur du tube définit la première lumière (440) ; et
(ii) une gaine (108) surmoulée autour du tube, dans laquelle une partie de l'extérieur du tube et une partie de la gaine définissent ensemble la troisième lumière (452).

5. Sonde de biopsie (102 ; 103) selon la revendication 4, dans laquelle la gaine (108) définit en outre la deuxième lumière (500) :

6. Sonde de biopsie (102 ; 103) selon la revendication 2, comprenant en outre un tube qui s'étend à travers la troisième lumière (452), dans laquelle le tube définit la deuxième lumière (500).

7. Sonde de biopsie (102 ; 103) selon la revendication 1, comprenant en outre un raccord (476) fixe par rapport à la canule (12), dans laquelle le raccord comprend un premier orifice (520) en communication fluidique avec la deuxième lumière (500).

8. Sonde de biopsie (102 ; 103) selon la revendication 7, dans laquelle le premier orifice (620) comprend une cloison (524) que peut pénétrer une aiguille.

9. Sonde de biopsie (102 ; 103) selon la revendication 7, dans laquelle le raccord (476) comprend en outre un second orifice (484) en communication fluidique avec au moins une partie de la canule (12).

10. Sonde de biopsie (102 ; 103) selon la revendication 9, dans laquelle le premier orifice (520) et le second orifice (484) sont isolés de manière fluidique l'un de l'autre.

11. Sonde de biopsie (102 ; 103) selon la revendication 7, comprenant en outre un accouplement (522) entre le premier orifice (520) et la deuxième lumière (500).

12. Sonde de biopsie (102 ; 103) selon la revendication 7, dans laquelle la canule (12) définit un axe, dans laquelle le premier orifice (520) s'étend de manière transversale par rapport à l'axe.

13. Sonde de biopsie (102 ; 103) selon la revendication 2, dans laquelle la troisième lumière (452) est sensiblement parallèle à la première lumière (440).
